# EUROPÄISCHE PATENTSCHRIFT

(11) **EP 1 453 531 B1**
(45) Veröffentlichungstag und Bekanntmachung des Hinweises auf die Patenterteilung: **14.05.2008**
(21) Anmeldenummer: 02793089.0
(22) Anmeldetag: 19.12.2002
(51) Int. Cl.: G01N 33/68

(54) **VERWENDUNG VON HMGB-PROTEINEN UND DAFÜR CODIERENDEN NUKLEINSÄUREN**
USE OF HMGB PROTEINS AND NUCLEIC ACIDS THAT CODE THEREFOR
UTILISATION DE PROTEINES HMGB ET D'ACIDES NUCLEIQUES CODANT POUR CELLES-CI

(30) Priorität: 19.12.2001 DE 10162556
(43) Veröffentlichungstag der Anmeldung: 08.09.2004
(73) Patentinhaber: Alcedo Biotech GmbH, 28359 Bremen (DE)
(72) Erfinder: BULLERDIEK, Jörn, 28357 Bremen (DE)
(74) Vertreter: Bohmann, Armin K.
(86) Internationale Anmeldenummer: PCT/EP2002/014579
(87) Internationale Veröffentlichungsnummer: WO 2003/051383

(56) Entgegenhaltungen:
- EP-A- 0 727 487
- WO-A1-99/07856
- WO-A2-99/63115
- DE-A- 19 548 122
- US-A- 6 165 737
- DATABASE WPI Section Ch, Week 199851 Derwent Publications Ltd., London, GB; Class B02, AN 1998-610380 XP002252337 & WO 98 50536 A (UNIV NEW JERSEY MEDICINE & DENTISTRY), 12. November 1998 (1998-11-12)
- TAGUCHI AKIHIKO ET AL: "Blockade of RAGE-amphoterin signalling suppresses tumour growth and metastases." NATURE (LONDON), Bd. 405, Nr. 6784, 2000, Seiten 354-360, XP002252336 ISSN: 0028-0836 in der Anmeldung erwähnt
- CZURA C.J. ET AL JOURNAL OF ENDOTOXIN RESEARCH Bd. 7, Nr. 4, 2001, Seiten 315 - 321
- WANG H. ET AL AMERICAN JOURNAL OF RESPIRATORY AND CRITICAL CARE MEIDCINE Bd. 164, 15 November 2001, Seiten 1768 - 1773
- LESSEY B.A. SEMINARS IN REPRODUCTIVE ENDOCRINOLOGY Bd. 15, Nr. 3, 1997, Seiten 291 - 299
- RONFANI L. ET AL DEVELOPMENT Bd. 128, April 2001, Seiten 1265 - 1273
- DATABASE PUBMED [Online] NCBI PMID: 10711829, 2000 MCLAREN J.
- DATABASE PUBMED [Online] PMID: 16365390, Januar 2006 MITOLA S. ET AL
- DATABASE PUBMED [Online] PMID: 15793304, 2005 SCHLUETER C. ET AL
- DATABASE [Online] Retrieved from HTTP://ALLP.COM/DRUG_DEV.HTM
- DATABASE [Online] Retrieved from HTTP://EN.WIKIPEDIA.ORG/WIKI/DRUG_DEVELOPME NT
- DATABASE [Online] Retrieved from HTTP://FDA.GOV/CDER/HANDBOOK/DEVELOP.HTM
- DATABASE [Online] Retrieved from HTTP://WWW.PPDI.COM/CORPORATE/FAQ/ABOUT_DRU G_DEVELOPMENT/HOME.HTM

### SEQUENZPROTOKOLL

<110> alcedo biotech GmbH
<120> Verwendung von HMGB-Proteinen und dafür codierenden Nukleinsäuren
<130> B 10017 PCT
<160> 11
<170> PatentIn version 3.1
<210> 1
   <211> 1207
   <212> DNA
   <213> Homo sapiens
<220>
   <221> misc_feature
   <222> (1)..(1207)
   <223> cDNA-Sequenz des HMGB1
<220>
   <221> CDS
   <222> (77)..(724)
   <223> Protein-codierende Sequenz (Accession-Nr.: BC003378)
<400> 1
   gagacagcgc cggggcaagt gagagccgga cgggcactgg gcgactctgt gcctcgctga      60
   ggaaaaataa ctaaac atg ggc aaa gga gat cct aag aag ccg aga ggc aaa     112
                     Met Gly Lys Gly Asp Pro Lys Lys Pro Arg Gly Lys
                     1               5                   10
   atg tca tca tat gca ttt ttt gtg caa act tgt cgg gag gag cat aag       160
   Met Ser Ser Tyr Ala Phe Phe Val Gln Thr Cys Arg Glu Glu His Lys
           15                  20                  25
   aag aag cac cca gat gct tca gtc aac ttc tca gag ttt tct aag aag       208
   Lys Lys His Pro Asp Ala Ser Val Asn Phe Ser Glu Phe Ser Lys Lys
       30                  35                  40
   tgc tca gag agg tgg aag acc atg tct gct aaa gag aaa gga aaa ttt       256
   Cys Ser Glu Arg Trp Lys Thr Met Ser Ala Lys Glu Lys Gly Lys Phe
   45                  50                  55                  60
   gaa gat atg gca aag gcg gac aag gcc cgt tat gaa aga gaa atg aaa       304
   Glu Asp Met Ala Lys Ala Asp Lys Ala Arg Tyr Glu Arg Glu Met Lys
                   65                  70                  75
   acc tat atc cct ccc aaa ggg gag aca aaa aag aag ttc aag gat ccc       352
   Thr Tyr Ile Pro Pro Lys Gly Glu Thr Lys Lys Lys Phe Lys Asp Pro
               80                  85                  90
   aat gca ccc aag agg cct cct tcg gcc ttc ttc ctc ttc tgc tct gag       400
   Asn Ala Pro Lys Arg Pro Pro Ser Ala Phe Phe Leu Phe Cys Ser Glu
           95                  100                 105
   tat cgc cca aaa atc aaa gga gaa cat cct ggc ctg tcc att ggt gat       448
   Tyr Arg Pro Lys Ile Lys Gly Glu His Pro Gly Leu Ser Ile Gly Asp
       110                 115                 120
   gtt gcg aag aaa ctg gga gag atg tgg aat aac act gct gca gat gac      496
   Val Ala Lys Lys Leu Gly Glu Met Trp Asn Asn Thr Ala Ala Asp Asp
   125                 130                 135                 140
   aag cag cct tat gaa aag aag gct gcg aag ctg aag gaa aaa tac gaa      544
   Lys Gln Pro Tyr Glu Lys Lys Ala Ala Lys Leu Lys Glu Lys Tyr Glu
                   145                 150                 155
   aag gat att gct gca tat cga gct aaa gga aag cct gat gca gca aaa      592
   Lys Asp Ile Ala Ala Tyr Arg Ala Lys Gly Lys Pro Asp Ala Ala Lys
               160                 165                 170
   aag gga gtt gtc aag gct gaa aaa agc aag aaa aag aag gaa gag gag      640
   Lys Gly Val Val Lys Ala Glu Lys Ser Lys Lys Lys Lys Glu Glu Glu
           175                 180                 185
   gaa gat gag gaa gat gaa gag gat gag gag gag gag gaa gat gaa gaa      688
   Glu Asp Glu Glu Asp Glu Glu Asp Glu Glu Glu Glu Glu Asp Glu Glu
       190                 195                 200
   gat gaa gat gaa gaa gaa gat gat gat gat gaa taa gttggttcta           734
   Asp Glu Asp Glu Glu Glu Asp Asp Asp Asp Glu
   205                 210                 215
   gcgcagtttt tttttcttgt ctataaagca tttaaccccc ctgtacacaa ctcactcctt    794
   ttaaagaaaa aaattgaaat gtaaggctgt gtaagatttg tttttaaact gtacagtgtc    854
   tttttttgta tagttaacac actaccgaat gtgtctttag atagccctgt cctggtggta   914
   ttttcaatag ccactaacct tgcctggtac agtatggggg ttgtaaattg gcatggaaat    974
   ttaaagcagg ttcttgttgg tgcacagcac aaattagtta tatatgggga tggtagtttt   1034
   ttcatcttca gttgtctctg atgcagctta tacgaaataa ttgttgttct gttaactgaa   1094
   taccactctg taattgcaaa aaaaaaaaaa gttgcagctg ttttgttgac attctgaatg   1154
   cttctaagta aatacaattt tttttattaa aaaaaaaaaa aaaaaaaaaa aaa          1207
<210> 2
   <211> 215
   <212> PRT
   <213> Homo sapiens
<220>
   <221> misc_feature
   <222> (1)..(1207)
   <223> cDNA-Sequenz des HMGB1
<400> 2
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Glu Asp Glu Glu
               180                 185                 190
   Asp Glu Glu Asp Glu Glu Glu Glu Glu Asp Glu Glu Asp Glu Asp Glu
           195                 200                 205
   Glu Glu Asp Asp Asp Asp Glu
       210                 215
<210> 3
   <211> 215
   <212> PRT
   <213> Homo sapiens
<220>
   <221> MISC_FEATURE
   <222> (1)..(215)
   <223> Aminosäuresequenz des HMGB1-Proteins (Accession-Nr.: S02826)
<400> 3
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Glu Asp Glu Glu
               180                 185                 190
   Asp Glu Glu Asp Glu Glu Glu Glu Glu Asp Glu Glu Asp Glu Asp Glu
           195                 200                 205
   Glu Glu Asp Asp Asp Asp Glu
       210                 215
<210> 4
   <211> 653
   <212> DNA
   <213> Homo sapiens
<220>
   <221> misc_feature
   <223> zusätzliches Transkript RAGE1 (cDNA)
<400> 4
   gatccccgtc ccaccttctc ctgtagcttc agcccaggcc ttccccgaca ccgggccttg     60
   cgcacagccc ccatccagcc ccgtgtctgg ggtgagcata ggtggggagg gccccaagct    120
   cacgtgagca cgttctggaa gtctgaccct tagggaaaga gggagtcaag cccatggcca    180
   ctgggatcac tcacaggtgt aactctccac ctcaaaaccc ttccaactcc cagagcctgt    240
   gcctctggag gaggtccaat cggtggtgga gccagaaggt ggagcagtag ctcctggtgg    300
   aaccgtaacc ctgacctgtg aagtccctgc ccagccctct cctcaaatcc actggatgaa    360
   ggatggtgtg cccttgcccc ttccccccag ccctgtgctg atcctccctg agatagggcc    420
   tcaggaccag ggaacctaca gctgtgtggc cacccattcc agccacgggc cccaggaaag    480
   ccgtgctgtc agcatcagca tcatcgaacc aggcgaggag gggccaactg caggtgaggg    540
   gtttgataaa gtcagggaag cagaagatag cccccaacac atgtgactgg ggggatggtc    600
   aacaagaaag gaatggaagg ccccagaaaa ccaggaggaa gaggaggagc gtg            653
<210> 5
   <211> 511
   <212> DNA
   <213> Homo sapiens
<220>
   <221> misc_feature
   <223> zusätzliches Transkript RAGE2 (cDNA)
<400> 5
   gatccccgtc ccaccttctc ctgtagcttc agcccaggcc ttccccgaca ccgggccttg     60
   cgcacagccc ccatccagcc ccgtgtctgg gaacctgtgc ctctggagga ggtccaattg    120
   gtggtggagc cagaaggtgg agcagtagct cctggtggaa ccgtaaccct gacctgtgaa    180
   gtccctgccc agccctctcc tcaaatccac tggatgaagg atggtgtgcc cttgcccctt    240
   ccccccagcc ctgtgctgat cctccccgag atagggcctc aggaccaggg aacctacagc    300
   tgtgtggcca cccattccag ccacgggccc caggaaagcc gtgctgtcag catcagcatc    360
   atcgaaccag gcgaggaggg gccaactgca ggtgaggggt ttgataaagt cagggaagca    420
   gaagatagcc cccaacacat gtgactgggg ggatggtcaa caagaaagga atggaaggcc    480
   ccagaaaacc aggaggaaga ggaggagcgt g                                   511
<210> 6
   <211> 698
   <212> DNA
   <213> Homo sapiens
<220>
   <221> misc_feature
   <223> zusätzliches Transkript RAGE3 (cDNA)
<400> 6
   gatccccgtc ccaccttctc ctgtagcttc agcccaggcc ttccccgaca ccgggccttg      60
   cgcacagccc ccatccagcc ccgtgtctgg ggtgagcata ggtggggagg gccccaagct     120
   cacgtgagca cgttctggaa gtctgaccct tagggaaaga gggagtcaag cccatggcca     180
   ctgggatcac tcacaagtgt aactctccac ctcaaaaccc ttccaactcc cagagcctgt     240
   gcctctggag gaggtccaat tggtggtgga gccagaaggt ggagcagtag ctcctggtgg     300
   aaccgtaacc ctgacctgtg aagtccctgc ccagccctct cctcaaatcc actggatgaa     360
   ggatggtgtg cccttgcccc ttccccccag ccctgtgctg atcctccctg agatagggcc     420
   tcaggaccag ggaacctaca gctgtgtggc cacccattcc agccacgggc cccaggaaag     480
   ccgtgctgtc agcatcagca tcatcgaacc aggcgaggag gggccaactg caggctctgt     540
   gggaggatca gggctgggaa ctctagccct ggccctgggg atcctgggag gcctggggac     600
   agccgccctg ctcattgggg tcatcttgtg gcaaaggcgg caacgccgag gagaggagag     660
   gaaggcccca gaaaaccagg aggaagagga ggagcgtg                             698
<210> 7
   <211> 23
   <212> DNA
   <213> Artificial Sequence
<220>
   <223> Sequenzierprimer (Laborcode: M13uni)
<400> 7
   cccagtcacg acgttgtaaa acg 23
<210> 8
   <211> 23
   <212> DNA
   <213> Artificial Sequence
<220>
   <223> Sequenzierprimer (Laborcode: M13rev)
<400> 8
   agcggataac aatttcacac agg 23
<210> 9
   <211> 34
   <212> DNA
   <213> Artificial Sequence
<220>
   <223> Adaptionsprimer (Laborcode: AP2)
<400> 9
   aaggatccgt cgacatcttt tttttttttt tttt 34
<210> 10
   <211> 27
   <212> DNA
   <213> Artificial Sequence
<220>
   <223> Detektionsprimer (Laborcode: PrimerRAGE2up)
<400> 10
   gatccccgtc ccaccttctc ctgtagc 27
<210> 11
   <211> 30
   <212> DNA
   <213> Artificial Sequence
<220>
   <223> Detektionsprimer (Laborcode: RAGE21o)
<400> 11
   cacgctcctc ctcttcctcc tggttttctg 30

## Beschreibung

Die vorliegende Erfindung betrifft die Verwendung von HMGB, von einer dafür codierenden Nukleinsäure, von einem insbesondere natürlichen Wechselwirkungspartner von HMGB und/oder von einer dafür codierenden Nukleinsäure als Zielmolekül für die Entwicklung eines Medikamentes und/oder eines diagnostischen Mittels, die Verwendung von mit HMGB, von mit einer dafür codierenden Nukleinsäure, von mit einem insbesondere natürlichen Wechselwirkungspartner von HMGB und/oder von mit einer dafür codierenden Nukleinsäure in Wechselwirkung tretenden chemischen Verbindungen zur Herstellung und/oder Entwicklung eines Medikamentes und/oder diagnostischen Mittels.

Die High-Mobility-Group-Proteine sind kleine, Chromatin-assoziierte Nichtlüstonproteine, die u.a. aufgrund ihrer funktionellen Sequenzmotive in drei Familien unterteilt werden: die HMGB-Familie, die HMGN-Familie und die HMGA-Familie. (Bustin M., Revised nomenclature for high mobility group (HMG) chromosomal proteins. Trends Biochem. Sci. 2001, 26:152-1533). Die Zuordnung zu einer Familie erfolgt aufgrund der DNA-Bidungsdomänen. Die Proteine der HMGB-Familie besitzen sog. DNA-Boxen als Bindungsdomänen. In der Abkürzung des Familiennamens steht insoweit der Buchstabe B für Box. HMGB1 ist das am besten untersuchte Protein der HMGB-Familie, zu der neben weiteren Proteinen noch HMGB2, HMGB3 und SP100-HMG gehören.

Im fortpflanzungsfähigen Alter unterliegt das im *cavum uteri* befindliche Endometrium zyklischen Veränderungen, welche durch Hormone des Ovars induziert werden. Liegen an anderen Stellen als dem Uterus-Innenraum Endometriumherde vor, so wird von einer Endometriose gesprochen. Die Endometriose kommt bei ca. 10-15% der Frauen im geschlechtsreifen Alter (ca. 20. - 40. Lebensjahr) vor. Aufgrund der Symptomatik wie Sterilität, starke Schmerzen, Blutungen und erhöhter Abortrate ist die Endometriose von außerordentlicher klinischer Relevanz (Baltzer, J. und Mickan, H. (1994): Gynäkologie - Ein kurzgefasstes Lehrbuch. Thieme, Stuttgart, S. 206-214) (Gogusev, J. Bouquet de Joliniere, J., Telvi, L., Doussau, M., du Manoir, S., Stoikoski, A., Levardon M. (1999): Detection of DNA copy number changes in human endometriosis by comparative genomic hybridization. Hum Genet 105: 444-451). Mögliche Therapien basieren auf Therapien zur Veränderung des Hormonhaushaltes mit Hormonantagonisten, sowie auf chirurgischen Eingriffen (Laparoskopie oder Laparotomie) zur Entfernung endometriotischer Herde bei möglicher Organerhaltung (Schmidt-Matthiesen, H. und Hepp, H. (1998): Gynäkologie und Geburtshilfe. Schattauer, Stuttgart, S. 339-340). Nachteilig bei den vorstehend beschriebenen Therapiemöglichkeiten wirkt sich dabei aus, dass keine dieser Therapien kausal ist

Der vorliegenden Erfindung liegt somit die Aufgabe zugrunde, Mittel bereitzustellen, die eine kausale Therapie von zum Formenkreis der Endometriose gehörenden Erkrankungen ermöglichen. Weiterhin ist es eine der vorliegenden Erfindung zugrundeliegenden Aufgabe, ein Mittel bereitzustellen, welches die Entwicklung von Medikamenten zur Behandlung von Endometriose und allgemein Erkrankungen des Endometriums erlauben. Schließlich ist es eine Aufgabe der vorliegenden Erfindung, Mittel zur Diagnose von Erkrankungen des Endometriums bereitzustellen.

Erfindungsgemäß wird die Aufgabe gelöst durch den Gegenstand der unabhängigen Ansprüche. Bevorzugte Ausführungsformen ergeben sich aus den Unteransprüchen.

Der vorliegenden Erfindung liegt die überraschende Erkenntnis zugrunde, dass entgegen der bisher im Stand der Technik verbreiteten Auffassung, dass HMGB-Proteine wie HMGB1, HMGB2, HMGB3 und SP100-HMG, und insbesondere HMGB1, ubiquitär in allen Zellen in gleicher Konzentration vorhanden sind, nicht zutreffend ist. Vielmehr haben die vorliegenden Erfinder festgestellt, dass das Endometrium eine besonders starke Expression von HMGB und insbesondere HMGB1 zeigt. Darüber hinaus wurde seitens der vorliegenden Erfinder gefunden, dass sich der Titer an HMGB und insbesondere HMGB1 während des endometrialen Zyklusses signifikant verändert. Schließlich haben die vorliegenden Erfinder gefunden, dass die Menge bzw. Konzentration von RAGE, mithin eines Wechselwirkungspartners von HMG und insbesondere HMGB1, während des endometrialen Zykluses sich ebenfalls ändert. Ohne im Folgenden darauf festgelegt sein zu wollen ergibt sich damit die Möglichkeit, durch Blockieren von HMGB 1 bzw. seines Rezeptors, die normalerweise beobachtete Wirkung von HMGB1 zu unterbinden. Eine Unterbindung der Wirkungen kann entsprechend in einem therapeutischen Konzept verwendet werden, bei dem die durch HMGB, insbesondere HMBG1 und seinem Wechselwirkungspartner, insbesondere RAGE, vermittelten Wirkungen unterbunden werden. Dies kann im Rahmen der hierin beschriebenen Erkrankungen des Endometriums in vorteilhafter Weise, d. h. zur Prävention und zur Behandlung verwendet werden. Darüber hinaus sind die HMG-Proteine sowie deren Wechselwirkungspartner, insbesondere HMGB1 bzw. RAGE, somit geeignete Marker zum Monitoren des Zustandes des Endometriums bzw. von Erkrankungen des Endometriums, wie sie hierin offenbart sind. Dabei wird bevorzugterweise durch eine Verringerung des biologisch aktiven HMGB bzw. seines Wechselwirkungspartners, wie insbesondere RAGE, die jeweilige Erkrankung diagnostiziert, therapiert, in ihrer Progression beeinflusst, bevorzugterweise verlangsamt, bzw. der Therapieerfolg verfolgt.

Auf der Grundlage dieser überraschenden Einsichten in die Bedeutung von HMGB und seiner Wechselwirkungspartner, insbesondere RAGE, im endometrialen Geschehen gründen sich die verschiedenen Aspekte der vorliegenden Erfindung.

Unter dem Begriff HMGB sollen hierin alle Proteine der HMGB-Familie verstanden werden. Insoweit betrifft die vorliegende Erfindung die Verwendungen der HMGB-Proteine sowie der für sie codierenden Nukleinsäuren. Insbesondere betrifft die Erfindung dabei in ihren verschiedenen hierin offenbarten Aspekten die Verwendung von HMGB1, HMGB2, HMGB3 und SP100-HMG und ganz besonderes HMGB1 bzw. der für sie codierenden Nukleinsäuren. Unter HMGB sollen dabei auch solche HMGB-Moleküle verstanden werden, die beispielsweise Deletionen, Mutationen und Modifikationen aufweisen, und die hierin allgemein als modifizierte HMGB-Proteine bezeichnet werden. Ein HMGB-Protein im Sinne der vorliegenden Erfindung wird so lange als HMGB-Protein betrachtet, wie es zumindest eine der Eigenschaftender der nicht-modifizierten Form zeigt oder aufweist. Entsprechende Modifikationen sind im Rahmen des Könnens der Fachleute auf diesem Gebiet.

Die vorliegende Erfindung wird im folgenden anhand von HMGB1 beispielhaft erläutert.

HMGB1 gehört zur Gruppe High-Mobility-Group-Proteine. HMGB1 ist das am besten untersuchte Protein der HMGB-Familie. Es sind grundsätzlich zwei verschiedene Funktionen des HMGB1 bekannt. Zum einen beeinflusst es im Zellkern als sogenannter architektonischer Transkriptionsfaktor die Entstehung von Transkriptionsfaktorkomplexen für bestimmte Zielgene, so z.B. die Bindung des Östrogenrezeptors an spezifische DNA-Sequenzen (Boonyaratanakornkit, V., Melvin V., Prendergast, P., Altmann, M., Ronfani, L., Bianchi, M.E., Tarasevicience, L., Nordeen, S.K., Allegretto, E.A., Edwards, D.P. (1998): High-mobility group chromatin proteins 1 and 2 functionally interact with steroid hormone receptors to enhance their DANN binding in vitro and transcriptional activity in mammalian cells. Mol Cell Biol 18: 4471-4487). Zum anderen ist HMGB1 extrazellulär ein Ligand des Zelloberflächenrezeptors RAGE (receptor for advanced glycation end products) (Taguchi, A., Blood, D.C., del Toro, G., Canet, A., Lee, D.C., Qu, W., Tanji, N., Lu, Y., Lalla, E., Fu, C., Hofmann, M.A., Kislinger, T., Ingram, M., Lu, A., Tanaka, H., Hori, O., Ogawa, S. Stern, D.M., Schmidt, A.M. (2000): Blockade of RAGE-amphetorin signalling suppresses tumour growth and metastases. Nature 405: 354-360) und somit RAGE ein Wechselwirkungspartner vom HMGB im Sinne der vorliegenden Erfindung. In dieser Funktion ist HMGB1 bei der Tumormetastasierung beteiligt und es spielt eine Rolle bei zentralen zellulären Signalübertragungswegen wie z.B. p21ras, MAP-Kinase, NF-kB und cdc42/rac. Die Fähigkeit HMGB1 zu sezernieren ist nur auf bestimmte Zelltypen beschränkt: Makrophagen, Neuronen und spezifische Tumorzellinien (Taguchi, A., Blood, D.C., del Toro, G., Canet, A., Lee, D.C., Qu, W., Tanji, N., Lu, Y., Lalla, E., Fu, C., Hofmann, M.A., Kislinger, T., Ingram, M., Lu, A., Tanaka, H., Hori, O., Ogawa, S. Stern, D.M., Schmidt, A.M. (2000): Blockade of RAGE-amphetorin signalling suppresses tumour growth and metastases. Nature 405: 354-360) und (Müller, S., Scaffidi, P., Degryse, B., Bonaldi, T., Ronfani, L., Agresti, A., Beltrame, M., Bianchi, M.E., (2001): New EMBO members' review: the double life of HMGB1 chromatin protein: architectural factor and extracellular signal. EMBO J 20: 4337-4340).

Bei der Endometriose handelt es sich um einen ausgesprochen komplexen Vorgang mit einer nach wie vor unklaren Pathogenese. Histologisch zeigen sich endometriale Drüsen, welche von Stroma umgeben sind (Thomas, C. (1998): Histopathologie. Schattauer, Stuttgart, S. 249-250). Es handelt sich daher um eine Gewebsveränderung die sowohl aus Epithel als auch Mesenchym besteht. Bei der Endometriose handelt es sich nach der gültigen Vorstellung um eine Schleimhautektopie.

Darüber hinaus wird in Abhängigkeit von der Lokalisation die Endometriose diese in verschiedene Gruppen unterteilt (Baltzer, J. und Mickan, H. (1994): Gynäkologie - Ein kurzgefasstes Lehrbuch. Thieme, Stuttgart, S. 206-214), die hierin allgemein als Endometriose bezeichnet werden:
1. Endometriosis genitalis interna, vorwiegend Uteruswand (Adenomyosis uteri) und Tube
2. Endometriosis genitalis externa, vorwiegend retrozervikales Douglas-Peritoneum, Ovar und Portio
3. Endometriosis extragenitalis, vorwiegend Harnblase, Operationsnarben, Pleura, Ureter und Darm.

Diese ektopen Schleimhautinseln nehmen größtenteils unter dem Einfluß von Ovarialhormonen am Zyklus teil und zeigen entsprechend periodische Veränderungen, d.h. Schleimhautproliferation, Zeichen der sekretorischen Phase, prämenstruelles Ödem und Schleimhautzerfall sowie Blutaustritt.

Die hierbei entstehenden Veränderungen führen abhängig von Lokalisation und Ausdehnung der Herde zu typischen Schmerzen. Typisch ist ferner ein prämenstruelles Einsetzen der Schmerzen und Nachlassen der Menstruation. Aufgrund der fehlenden Abflussmöglichkeiten für das zerfallende Gewebe und der Blutung kann es zu zystischen Organauftreibungen und Verwachsungen kommen, die wiederum Dauerschmerzen auslösen können (SCHMIDT-MATTHIESEN, H. UND HEPP, H. (1998): Gynäkologie und Geburtshilfe. Schattauer, Stuttgart, S. 339-340)

Zur Pathogenese der Endometriose existieren zahlreiche unterschiedliche Hypothesen (Baltzer, J. und Mickan, H. (1994): Gynäkologie - Ein kurzgefasstes Lehrbuch. Thieme, Stuttgart, S. 206-214) (Scheider, A. (2001): Frauenheilkunde. Auszug aus der Hauptvorlesung. Universität Jena. www.unijena.de/ufk/cd/endomose.htm), von denen die mit der größten Plausibilität die folgenden sind, wenngleich derzeit eine abschließende Beurteilung nicht möglich zu sein scheint und möglicherweise mehrere Faktoren an der Pathogenese beteiligt sein können:
- "de novo" (Verschleppung von Endometrium-Gewebe an ortsfremde Lokalisationen). Dabei kann die Verschleppung wiederum auf unterschiedlichem Wege stattfinden, nämlich durch retrograde Menstruation, vaskuläre Ausbreitung, mechanische Verschleppung (durch chirurgische Eingriffe), durch Invasion des Endometriums in das Myometrium, durch Sog infolge präovulatorischer utero-tubarer Peristaltik (Schokoladenzysten können entstehen).
- "in situ" (Umwandlung von multizentrischem Epithel in Endometriumgewebe): Embryonales Restgewebe (Zölomepithel, Epithel aus den Resten der Müller'schen Gänge bzw. der Wolff'schen Gänge) wird durch Metaplasie in Endometriumgewebe transformiert.
- endometriuminduzierte Metaplasie: Durch retrograde Menstruation an das Peritoneum gelangte Endometriumanteile lösen eine Differenzierung der undifferenzierten mesenchymalen Zellen in endometriales Gewebe aus.

Infolge der hierin offenbarten erhöhten Expression von HMGB und insbesondere HMGB in endometrialem Gewebe kann vor der bisher im Stand der Technik vorherrschenden Auffassung Abstand genommen werden, dass die HMGB-Proteine, die für sie codierende Nukleinsäure, aber auch die Wechselwirkungspartner, insbesondere die natürlichen Wechselwirkungspartner, von HMGB und insbesondere HMGB1 keine Zielmoleküle oder Targets darstellen, welche für die Entwicklung von Medikamenten herangezogen werden können. Im Lichte der hierin gemachten Offenbarung sind diese jedoch vielmehr im höchsten Maße relevante Zielmoleküle für die Herstellung oder Entwicklung eines Medikamentes oder eines Diagnostikums betreffend endometrisches Gewebe und Erkrankungen des Endometriums im speziellen. Erkrankungen des Endometriums, wie hierin verwendet, bezeichnet Endometriose in den verschiedenen, hierin beschriebenen Ausführunge.

Die Verwendung der HMGB-Proteine, der für sie codierenden Nukleinsäuren, der HMGB-Wechselwirkungspartner, insbesondere der natürlichen HMGB-Wechselwirkungspartner, und/oder der für sie codierenden Nukleinsäuren als Target kann dabei im Rahmen eines jeden Herstellungs- bzw. Entwicklungsprozesses für ein Medikament bzw. diagnostisches Mittel verwendet werden.

Das Medikament oder das diagnostische Mittel kann dabei ein Antikörper, eine mit HMGB1 in Wechselwirkung tretendes Aptamer oder Spiegelmer oder aber auch eine Nukleinsäure sein, die mit einer für HMGB1 codierenden Nukleinsäure, beispielsweise mRNA oder cDNA in Wechselwirkung tritt wobei die Nukleinsäure ausgewählt ist aus der Gruppe bestehend aus antisense-Oligonukleotid, Ribozym und RNAi. Das Medikament oder das diagnostische Mittel kann dabei auch eine trunkierte bzw. lösliche Form des RAGE, das sog. sRAGE, ein Antikörper gegen RAGE, eine mit RAGE in Wechselwirkung tretendes Aptamer oder Spiegelmer, oder aber auch eine Nukleinsäure sein, die mit einer für FAGE codierenden Nukleinsäure, beispielsweise mRNA oder cDNA, in Wechselwirkung tritt, wobei die Nukleinsäure ausgewählt ist aus der Gruppe bestehend aus antisense-Oligonukleotid, Ribozym und RNAi wobei in einem jeden Fall, das Medikament bzw. diagnostische Mittel für die Behandlung, Prävention und/oder Diagnose der verschiedenen Erkrankungen, wie hierin offenbart, verwendet werden kann.

Dabei ist es im Rahmen der vorliegenden Erfindung, dass anstelle des RAGE, wie es natürlicherweise vorkommt, sei es von humanen oder von anderen Quellen, insbesondere anderen Säugetierquellen, auch trunkierte Formen bzw. lösliche Formen davon verwendet werden können, die im Stand der Technik ebenfalls bekannt sind. Darüber hinaus ist es im Rahmen der vorliegenden Erfindung, dass die verkürzten Formen des RAGE, wie hierin im Beispielsteil beschrieben und von den Nukleinsäuren gemäß SEQ ID NO. 4, 5 und 6 codiert, im Rahmen der vorliegenden Erfindung verwendet werden können.

Bei der Herstellung eines für HMGB1 spezifischen Antikörpers wird dabei nach den im Stand der Technik bekannten Verfahren vorgegangen, wie sie den Fachleuten auf dem Gebiet bekannt sind. Besonders bevorzugt ist dabei die Verwendung von monoklonalen Antikörpern, die gemäß dem Protokoll von Cäsar und Milstein und Weiterentwicklungen davon hergestellt werden können. Antikörper sind dabei auch Antikörperfragmente oder -derivate, wie bspw. Fab-Fragmente, Fc-Fragmente aber auch einzelsträngige Antikörper, solange diese generell in der Lage sind, HMGB spezifisch zu binden. Neben monoklonalem Antikörpern können auch polyklonale Antikörper verwendet werden. Bin polyklonaler Antikörper für die Grundlagenforschung, der grundsätzlich auch als Medikament verwendet werden könnte, ist bspw. der gegen HMGB1 gerichtete Antikörper sc-12523 (Santa Cruz Biotechnology, Santa Cruz, USA). Bevorzugterweise sind die verwendeten Antikörper humane oder humanisierte Antikörper. Das vorstehend zu HMGB1 Gesagte gilt sinngemäß auch Für HMGB, die Wechselwirkungspartner von HMGB, insbesondere HMGB1, wie beispielsweise RAGE.

Es ist auch im Umfang der vorliegenden Erfindung, dass HMGB-Proteine, für sie codierende Nukleinsäuren, HMGB natürliche Wechselwirkungspartner und/oder für sie codierende Nukleinsäuren als Zielmolekül für die Herstellung von Aptameren und Spiegelmeren verwendet werden, wobei dies dann direkt oder indirekt als Medikament verwendet werden.

Aptamere sind D-Nukleinsäuren, entweder einzelsträngig oder doppelsträngig, die spezifisch an ein Zielmolekül binden. Die Herstellung von Aptameren ist bspw. beschrieben im Europäischen Patent EP 0 533 838. Dabei wird wie folgt vorgegangen:

Bei dem Verfahren zur Erzeugung der Aptameren wird eine Mischung aus Nukleinsäuren, d. h. potentiellen Aptameren bereitgestellt, wobei eine jede Nukleinsäure aus einem Segment aus wenigstens acht aufeinanderfolgenden, randomisierten Nukleotiden besteht und diese Mischung mit dem Zielmolekül oder Target, im vorliegenden Falle somit mit HMGB-Proteinen, für sie codierenden Nukleinsäuren, HMGB-Wechselwirkungspartnern, insbesondere den natürlichen Wechselwirkungspartnern und/oder für sie codierenden Nukleinsäuren, in Kontakt gebracht wird, wobei Nukleinsäuren, die an das Target binden, ggf. auf der Grundlage einer erhöhten Affinität, verglichen mit der Kandidatenmischung, vom Rest der Kandidatenmischung abgetrennt werden und die solchermaßen erhaltenen an das Target, ggf. mit einer höheren Affinität oder Kraft, bindenden Nukleinsäuren amplifiziert werden. Diese Schritte werden mehrfach wiederholt, so dass am Ende des Verfahrens spezifisch an das jeweilige Target oder Zielmolekül bindende Nukleinsäuren, sogenannte Aptamere, erhalten werden. Es ist im Rahmen der vorliegenden Erfindung, dass diese Aptamere stabilisiert werden können, bspw. durch Einführung bestimmter chemischer Gruppen, wie den Fachleuten auf dem Gebiet der Aptamer-Entwicklung bekannt. Aptamere werden derzeit bereits therapeutisch eingesetzt. Es ist auch im Rahmen der vorliegenden Erfindung, dass die solchermaßen hergestellten Aptamere für die Targetvalidierung und/oder als Leitsubstanzen für die Entwicklung von Medikamenten, insbesondere von kleinen Molekülen, verwendet werden.

Auf einem grundsätzlich ähnlichen Prinzip beruht die Herstellung oder Erzeugung von Spiegelmeren, die im Rahmen der vorliegenden Erfindung auf der Grundlage der HMGB-Proteine, der für sie codierenden Nukleinsäuren, der HMGB natürlichen Wechselwirkungspartner, und/oder der für sie codierenden Nukleinsäuren als Zielmolekül entwickelt werden können. Die Herstellung von Spiegelmeren ist bspw. beschrieben in der internationalen Patentanmeldung WO 98/08856. Spiegelmere sind L-Nukleinsäure, d.h. bestehen aus L-Nukleotiden, und zeichnen sich im wesentlichen dadurch aus, dass sie in biologischen Systemen eine sehr hohe Stabilität aufweisen und gleichzeitig, vergleichbar den Aptameren, mit einem Zielmolekül spezifisch wechselwirken bzw. an dieses binden können. Bei der Herstellung der Spiegelmere wird genauer so vorgegangen, dass eine heterogene Population aus D-Nukleinsäuren erzeugt wird, die Population mit dem optischen Antipoden des Zielmoleküls in Kontakt wird, im vorliegenden Falle somit mit dem D-Enantiomer des natürlicher Weise auftretenden L-Enantiomers, sodann jene D-Nukleinsäuren abgetrennt werden, die nicht mit der optischen Antipode des Zielmoleküls in Wechselwirkung getreten sind, die D-Nukleinsäuren, die mit der optischen Antipode des Zielmoleküls in Wechselwirkung getreten sind, bestimmt, ggf. abgetrennt und sequenziert werden und anschließend L-Nukleinsäuren synthetisiert werden, die in ihrer Sequenz mit derjenigen der zuvor für die D-Nukleinsäuren ermittelten Sequenzen identisch sind. Ähnlich dem Verfahren zur Herstellung von Aptameren ist es auch hier möglich, durch mehrfaches Wiederholen der Schritte geeignete Nukleinsäuren, d.h. Spiegelmere anzureichern bzw. zu erzeugen.

Eine weitere Klasse von Verbindungen, die unter Verwendung von HMGB-Proteinen und Wechselwirkungspartnern davon bzw. der für diese codierenden Nukleinsäuren hergestellt bzw. entwickelt werden können, sind Ribozyme, Antisense-Oligonukleotide und RNAi.

All diesen Klassen ist dabei gemein, dass sie nicht auf der Ebene des Translationsproduktes, d.h. auf der Ebene der Proteine (HMGB-Proteine und Protein-Wechselwirkungspartner davon) ihre Wirkung entfalten, sondern auf der Ebene der für das jeweilige Protein codierenden Nukleinsäure, insbesondere der für HMGB1 codierenden mRNA.

Bei Ribozymen handelt es sich um katalytisch aktive Nukleinsäuren, die bevorzugter Weise aus RNA aufgebaut sind und aus zwei Teilbereichen bestehen. Der erste Teilbereich ist für eine katalytische Aktivität verantwortlich, wohingegen der zweite Teil für eine spezifische Wechselwirkung mit einer Ziel-Nukleinsäure verantwortlich ist. Kommt es zur Ausbildung einer Wechselwirkung zwischen der Ziel-Nukleinsäure und dem zweiten Teil des Ribozyms, typischer Weise durch Hybridisierung von zueinander im wesentlichen komplementären Basenbereichen, kann der katalytische Teil des Ribozyms entweder intramolekular oder intermolekular, wobei letzteres bevorzugt ist, die Ziel-Nukleinsäure hydrolysieren für den Fall, dass die katalytische Wirkung des Ribozyms eine Phosphodiesterase-Aktivität ist. In der Folge kommt es zu einem - ggf. weiteren - Abbau der codierenden Nukleinsäure, wobei der Titer des Zielmoleküls sowohl auf der Nukleinsäure- als auch der Proteinebene sowohl intrazellulär als auch extrazellulär verringert wird und somit eine therapeutischer Ansatz bei Erkrankungen des Endometriums bereitgestellt wird. Ribozyme, deren Verwendung sowie Konstruktionsprinzipien sind den Fachleuten auf dem Gebiet bekannt und bspw. beschrieben in Doherty und Doudna (Ribozyme structures and mechanisms. Annu Rev Biophys Biomol Struct 2001;30:457-75) und Lewin und Hauswirth (Ribozyme gene therapy: applications for molecular medicine. Trends Mol Med 2001, 7:221-8).

Auf einem grundsätzlich ähnlichen Wirkmechanismus beruht die Verwendung von Antisense-Oligonukleotiden zur Herstellung eines Medikamentes bzw. diagnostischen Mittels. Antisense-Oligonukleotide hybridisieren infolge Basenkomplementarität typischerweise mit einer Ziel-RNA, normaler Weise mit mRNA und aktivieren dadurch RNAaseH. RNAaseH wird sowohl durch Phosphodiester als auch Phosphorothioat-gekoppelte DNA aktiviert. Phosphodiestergekoppelte DNA wird jedoch schnell durch zelluläre Nukleasen mit Ausnahme von Phosphorothioat-gekoppelter DNA abgebaut. Diese resistenten, nicht-natürlicher Weise auftretende DNA-Derivate inhibieren RNAaseH nicht, wenn sie mit RNA hybridisiert sind. Mit anderen Worten, Antisense-Polynukleotide sind nur als DNA-RNA-Hybridkomplex wirksam. Beispiele für derartige Antisense-Oligonukleotide finden sich, u.a. in US-Patent US 5,849,902 oder US 5,989,912. Prinzipiell besteht das wesentliche Konzept der Antisense-Oligonukleotide darin, gegen bestimmte RNA eine komplementäre Nukleinsäure bereitzustellen. Mit anderen Worten, ausgehend von der Kenntnis der Nukleinsäuresequenz der HMGB-Proteine und/oder ihrer Wechselwirkungspartner, insbesondere der jeweiligen mRNA, können durch Basenkomplementarität geeignete Antisense-Oligonukleotide hergestellt werden, die zu einem Abbau der codierenden Nukleinsäure, insbesondere der mRNA, führen.

Eine weitere Klasse von Verbindungen, die als Medikament bzw. diagnostisches Mittel grundsätzlich und insbesondere auch für die Therapie und/oder Prävention bzw. Diagnose der hierin beschriebenen Erkrankungen des Endometriums geeignet wären, ist die sogenannte RNAi. RNAi ist eine doppelsträngige RNA, die RNA-Interferenz vermittelt und typischerweise eine Länge von etwa 21 bis 23 Nukleotiden aufweist. Dabei entspricht einer der beiden Stränge der RNA einer Sequenz eines abzubauenden Genes. Mit anderen Worten, ausgehend von der Kenntnis der für die HMGB und/oder deren Wechselwirkungspartner codierenden Nukleinsäure, insbesondere der mRNA, kann eine doppelsträngige RNA hergestellt werden, wobei einer der beiden RNA-Stränge komplementär zu der besagten, für die HMGB und/oder deren Wechselwirkungspartner codierenden Nukleinsäure ist, bevorzugter Weise zur mRNA, und diese dann zum Abbau der entsprechenden codierenden Nukleinsäure führt und damit einhergehend zu einer Verringerung des Titers der jeweiligen Proteine. Die Erzeugung und Verwendung von RNAi als Medikament bzw. diagnostisches Mittel ist bspw. beschrieben in den internationalen Patentanmeldungen WO 00/44895 und WO 01/75164.

Mit Blick auf die Wirkmechanismen der vorstehend beschriebenen Klassen, Ribozymen, Antisense-Oligonukleotiden sowie RNAi, ist es somit im Rahmen der vorliegenden Erfindung, neben den HMGB-Proteinen und deren insbesondere natürlichen Wechselwirkungspartnern auch die dafür codierenden Nukleinsäuren, insbesondere die mRNA, zur Herstellung eines Medikamentes für die Behandlung und/oder Prävention von Erkrankungen des Endometriums bzw. für die Herstellung eines diagnostischen Mittels für die Diagnose von endometrischen Erkrankungen und dem Überwachen des Verlaufes der Erkrankung bzw. der angesetzten Therapie, entweder direkt oder als Zielmolekül, zu verwenden.

Es ist weiter im Rahmen der vorliegenden Erfindung, dass die vorstehend genannten Klassen von Verbindungen, d.h. Antikörper, Aptamere, Spiegelmere, Ribozyme, Antisense-Oligonukleotide sowie RNAi für die Herstellung eines Medikamentes zur Behandlung von Erkrankungen des Endometriums verwendet werden können. Die solchermaßen hergestellten Verbindungen der verschiedenen Klassen können auch Gegenstand einer pharmazeutischen Zusammensetzung oder eines diagnostischen Mittels sein, welche bevorzugter Weise für die Behandlung von Erkrankungen des Endometriums verwendet wird. Die pharmazeutische Zusammensetzung umfasst in einer Ausführungsform neben einer oder mehreren der vorstehend genannten bzw., wie hierin offenbart, erzeugten Verbindungen noch andere pharmazeutisch wirksame Verbindungen, wie bspw. Steroidhormone sowie einen pharmazeutisch akzeptablen Träger. Derartige Träger können bspw. flüssig oder fest sein, bspw. eine Lösung, ein Puffer, eine alkoholische Lösung und dergleichen. Als geeignete feste Träger kommen bspw. Stärke und dergleichen in Betracht. Es ist den Fachleuten auf dem Gebiet der pharmazeutischen Darreichungsformen bekannt, wie die entsprechenden Verbindungen der verschiedenen Klassen formuliert werden müssen, um auf dem jeweils angestrebten Darreichungsweg, bspw. oral, parenteral, subcutan, intravenös und dergleichen mehr, verabreicht werden zu können.

Die verschiedenen Verbindungen der verschiedenen Klassen können auch einzeln oder gemeinsam Gegenstand eines Kits sein, wobei der Kit die Verbindung und ggf. noch ein oder mehrere weitere Elemente umfasst, die aus der Gruppe ausgewählt sind, die Puffer, Negativ-Kontrollen, Positiv-Kontrollen und Benutzungsanweisungen umfasst. Typischerweise sind die einzelnen Verbindungen in trockener oder flüssiger Form, bevorzugter Weise für den einzelnen Anwendungsfall portioniert, in dem Kit enthalten. Der Kit kann dabei bevorzugter Weise verwendet werden zur Bestimmung des Zustandes des Endometriums aufgrund der hierin offenbarten Korrelation zwischen Titer an HMGB-Proteinen und insbesondere HMGB1 und dem zeitlichen Verlauf des endometrialen Zyklusses. Dies beruht dabei auf der hierin offenbarten Beobachtung, dass Unterschiede in der HMGB- und speziell der HMGB1-Expression in der Zyklusphase auftreten können.

Im Zusammenhang mit den verschiedenen hierin offenbarten Klassen von Verbindungen, die als therapeutisches Mittel oder diagnostisches Mittel erfindungsgemäß verwendet werden können, ist dabei ein Aspekt der, dass bestimmte Klassen direkt mit den HMGB-Proteinen bzw. den insbesondere als Protein(e) vorliegenden Wechselwirkungspartner(n) der HMGB-Proteine in Wechselwirkung treten. Es ist jedoch auch im Rahmen der vorliegenden Erfindung, dass die besagten Verbindungen der verschiedenen Klassen, insbesondere wenn es sich dabei um Antikörper, Aptamere und Spiegelmere handelt, den Wechselwirkungspartner des HMGB durch eine mehr oder weniger spezifische Wechselwirkung für das HMGB blockieren. Insoweit ist der Begriff der Verwendung von HMGB hierin auch dahingehend zu verstehen, dass er die Verwendung eines oder mehrerer des/der HMGB-Wechselwirkungspartner wie bspw. Rezeptoren umfasst. Die hierin beschriebenen Verfahren sind dann insoweit zu modifizieren, als dass anstelle der HMGB-Proteine bzw. der für sie codierenden Nukleinsäuren ein Wechselwirkungspartner davon in den verschiedenen Selektionsverfahren, Assays, ScreeningVerfahren oder Herstellungsverfahren eingesetzt wird.

Ohne im folgenden darauf festgelegt sein zu wollen, scheint der Grund für die Wirksamkeit von gegen HMGB und insbesondere HMGB1 oder deren Wechselwirkungspartner(n) gerichteten Wirkstoffen in der Unterbindung der Ausbildung von Transkriptionsfaktor-Komplexen begründet zu sein bzw. bei jenen Erkrankungen des Endometriums, bei denen insbesondere HMGB1 als extrazellulärer Ligand vorliegt, dessen Wechselwirkung mit seinen Zielstrukturen oder die Zielstrukturen selbst zu blockieren.

Im Rahmen der vorliegenden Erfindung werden unter natürlichen Wechselwirkungspartnern der HMGB-Proteine, RAGE, sRAGE und ein Translationsprodukt einer Nukleinsäure gemäß SEQ ID NO. 4, 5 oder 6 bezeichnet.

Im Übrigen werden hierin die Begriffe Protein, Polypeptid und Peptid, sofern nichts gegenteiliges vermerkt, synonym verwendet.

Schließlich ist es im Rahmen der vorliegenden Erfindung, dass diese all jene Verbindungen betrifft, die durch die hierin beschriebenen Verfahren hergestellt, oder im Rahmen der verschiedenen beschriebenen Screeningverfahren erhalten werden können.

Für die hierin offenbarte Gruppe von HMGB-Proteinen, die HMGB1, HMGB2, HMGB3 und SP100-HMG umfasst, gelten die spezifischen Ergebnisse zu HMGB1 insoweit ganz besonders, als dass zwischen den verschiedenen Verbindungen eine sehr hohe Homologie besteht.

Die Erfindung wird nun anhand der folgenden Figuren und Beispiele erläutert, aus denen sich weitere Merkmale, Ausführungsformen und Vorteile der Erfindung ergeben. Dabei zeigt
- Fig. 1: den zeitlichen Zusammenhang zwischen HMGB1-Positivität im endometrialen Zyklus,
- Fig. 2: eine immunhistochemische Darstellung eines Endometriose-Areals unter Verwendung eines HMGB 1-spezifischen Antikörpers,

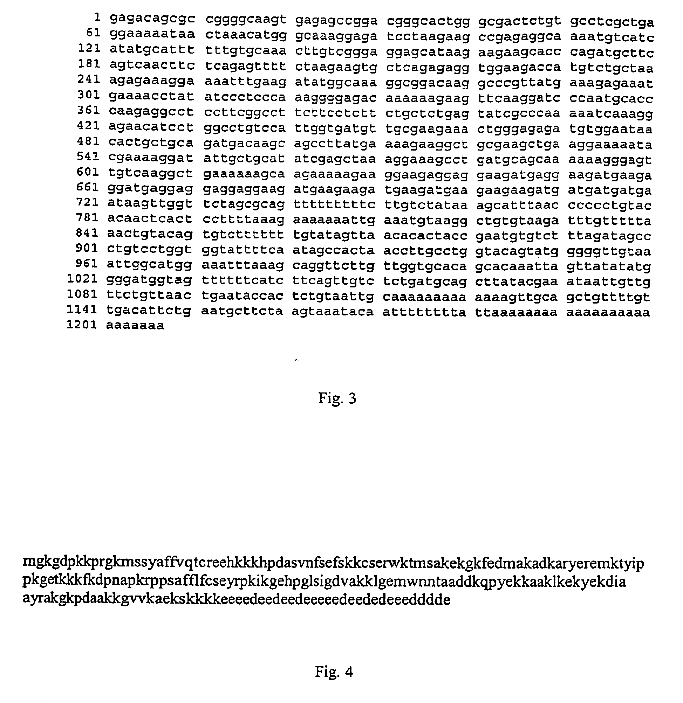

- Fig. 3: die cDNA-Sequenz des HMGB1-Gens, wobei die Protein-codierende Sequenz an Position 77 beginnt und an Position 724 endet (Zugangsnummer BC003378),
- Fig. 4: die Aminosäuresequenz des HMGB1-Proteins darstellt (Zugangsnummer S02826), und
- Fig. 5: ein Balkendiagramm, welches die Proliferationsrate der Endometriumkarzinomzelllinie Mz-12 nach Applikation unterschiedlicher HMGB1-Konzentrationen darstellt.

### Beispiel 1: Abhängigkeit der Expression von HMGB1-Protein von verschiedenen Zyklusphasen

Es wurden immunhistochemische Untersuchungen an Paraffinschnitten (5µm) von menschlichen Uterus-Endometrien und Endometriosen mit einem polyklonalen Antikörper (sc-12523, Santa Cruz Biotechnology, Santa Cruz, USA) aus der Ziege durchgeführt, der gegen ein Peptid aus einer internen Region des menschlichen HMGB1-Proteins gerichtet ist. Der verwendete Antikörper detektiert das HMGB1- und in schwächerem Maße das HMGB2-Protein.

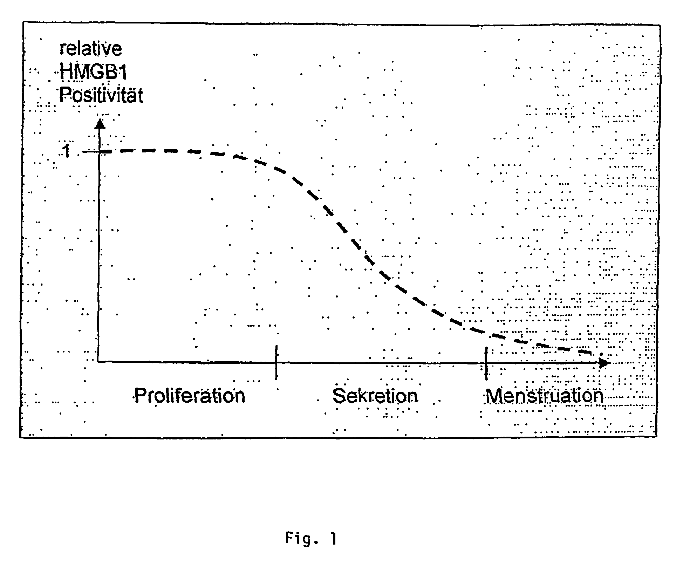

Das überraschende Ergebnis der immunhistochemischen Untersuchung an insgesamt 25 Gewebeschnitten mit normalem Endometrium und angrenzendem Myometrium in verschiedenen Zyklusphasen besteht darin, dass das HMGB1-Protein spezifisch nur in der Zytoplasmamembran des Drüsenepithels in der Proliferationsphase des Menstruationszyklus detektiert werden konnte. In den Sekretionsphasen nimmt die Positivität von HMGB1 in der Zytoplasmamembran insgesamt ab, ist aber in einigen Fällen noch nachweisbar (Fig.1). Die Bindegewebezellen des Endometriums und die glatten Muskelzellen des Myometriums wiesen in keiner Zyklusphase eine Immunreaktion auf.

### Beispiel 2: Immunhistochemischer Nachweis von Endometrioseherden

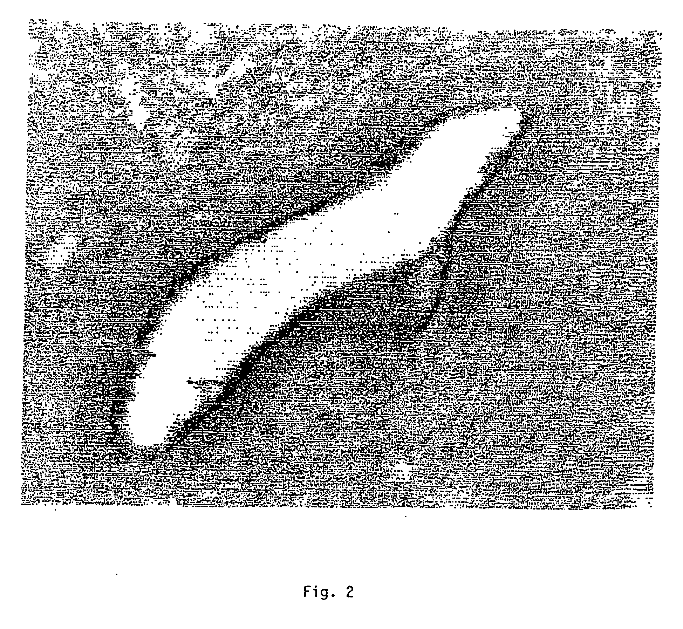

Es wurden immunhistochemische Untersuchungen an 20 Endometriose-Präparaten mit Hilfe des HMGB1-Antikörpers durchgeführt und festgestellt, dass Endometrioseherde überraschender Weise eine starke Posivität in der Zytoplasmamembran des Drüsenepithels zeigen, hingegen keine HMGB1-Positivität in den anderen Zellkompartimenten und dem umgebenden Gewebe. Exemplarisch ist ein Untersuchungsergebnis in Fig. 2 dargestellt, die einen Gewebeschnitt einer Adenomyose nach HMGB1-spezifischer Immunhistochemie zeigt. Dargestellt ist ein Ausschnitt einer Adenomyose (Adenomyosis uteri) bei einer Vergrößerung von 400.

### Beispiel 3: Erhöhte Zellproliferation durch HMGB1 Applikation in vitro

### METHODIK:

Die Messungen der Proliferationsrate erfolgte nach dem CellTiter 96^{R} AQᵤₑₒᵤₛ one solution cell proliferation assay der Firma Promega. Dabei wird mittels einer kolorimetrischen Messung das biologische Reduktionspotential von vitalen Zellen bestimmt.

Vorbereitend wurden Zellen der Endometriumkarzinomzelllinie Mz12 in RPMI 1640-Medium mit 10 %igem fetalen Kälberserum bei 37°C und 5 % CO₂ kultiviert, bis die Zellen eine konfluente Dichte erreichten. Nach der Trypsinierung der Zellen wurden diese in serumfreiem RPMI 1640-Medium aufgenommen und gleichmäßig auf 96-Well-Platten mit je 100 µl RPMI 1640-Medium (ohne fetales Kälberserum) pro Well verteilt und über Nacht bei 37°C und 5 % CO₂-Begasung inkubiert. Nachdem das Medium abgesaugt wurde, wurden pro Well 100 µl eines aus einer Verdünnungsreihe entstandenen Gemisches aus Medium und HMGB1-Protein mit einer jeweils definierten Konzentration des HMGB1-Proteins (1 ng/l 10 ng/l und 100 ng/l HMGB1) auf die Mz12-Zellen gegeben. Nach einer Inkubation von 24 h bei 37°C und 5 % CO₂ Begasung erfolgte die Zugabe von 20 µl CellTiter 96^{R} AQᵤₑₒᵤₛ one solution pro Well. Die Auswertung zur Bestimmung der Proliferationsrate erfolgte nach 1 h durch die Messung der Absorption bei 490 nm mittels eines Anthos-Reader, Modell 2001 der Firma Anthos.

### ERGEBNISSE:

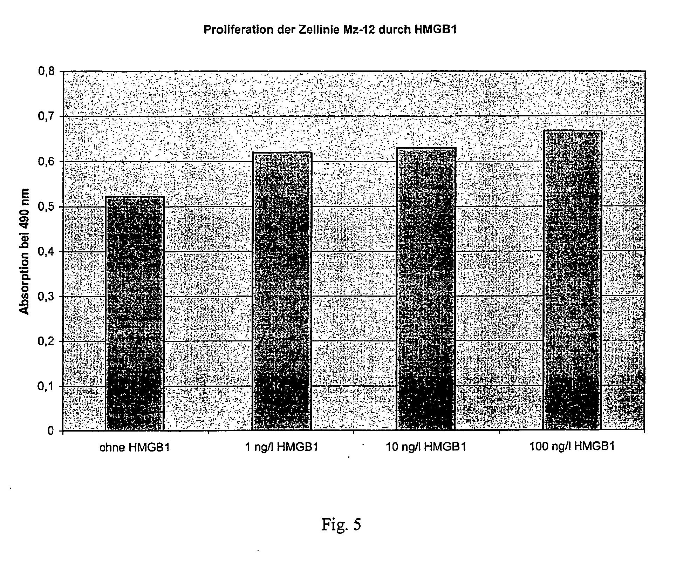

Im Vergleich zu den Zellen der Negativkontrolle, d. h. ohne HMGB Applikation, konnte bei den mit HMGB 1 behandelten Zellen eine signifikante Erhöhung der Proliferationsrate festgestellt werden. Zudem steht die Erhöhung der Proliferationsrate in Relation zur HMGB1-Konzentration, wie dies in Fig. 5 dargestellt ist.

### Beispiel 4: Markierung von HMGB1 mittels Fluorescein

### METHODIK:

Die Markierung von HMGB 1 erfolgte mit Hilfe des Fluorescein Labeling Kits der Firma Roche. Es wurden pro Ansatz 100 µg HMGB1 Protein lyophilisiert und in 100 µl PBS-Puffer resuspendiert. Zu dem gelösten HMGB1 wurden 1,5 µl FLUOS-Lösung (20 mg/ml), wobei FLUOS der Fluoreszenzfarbstoff des Kits zur Markierung von Proteinen ist und dabei die freie Aminogruppe des zu markierenden Proteins mit dem 5(6)-Carboxyfluorescein-N-hydroxysuccinimidester unter Bildung einer stabilen Amidbindung reagiert, gegeben und der Ansatz wurde lichtgeschützt unter Rühren 2 h bei RT inkubiert.

Zwischenzeitlich wurde die Sephadex-G-25-Säule mit 5 ml Blockierungslösung, die wie das PBS Bestandteil des entsprechenden Kits zunächst in Pulverform als Blockierungsreagenz, dann nach Anleitung mit bidest Wasser angesetzt wird, ist, und 30 ml PBS äquilibriert. Anschließend wurde der Reaktionsansatz auf die Säule übertragen und das markierte Protein mit 3,5 ml PBS eluiert. Das markierte Protein war jeweils in den ersten beiden Pools von jeweils 10 Tropfen (ca. 0,5 ml) enthalten.

### ERGEBNISSE:

Eine Verifizierung der Markierung erfolgte in der Reversed-Phase-HPLC unter Verwendung einer C18 Säule und eines binären Gradienten durch Ermittlung der veränderten Retensionszeit (Vergleich der Peaks von FLUOS-Lösung/markiertes HMGB1). Zusätzlich wurde das markierte HMGB1 in einem 12%igen PA-Gel unter denaturierenden Bedingungen aufgetrennt. Dabei zeigte das markierte Protein bei UV-Licht eine deutliche Bande, welches mittels Coomassie-Färbung bestätigt werden konnte. Es wurden ca. 60 µg HMGB1 in einem Endvolumen von 1ml mit Fluorescein markiert.

### Beispie15: Bindung von Fluorescein-markierten HMGb1-Protein an die Zellmembran von Zellen der Endometriumkarzinomzelllinie Mz-12

### METHODIK:

Die Mz-12-Zellen wurden vorbereitend in Leighton-tubes, d.h. speziellen Röhrchen zur Kultur von Zellen, mit je 1 ml RPMI 1640-Medium über Nacht bei 37°C und 5 % CO₂ inkubiert. Anschließend wurden 350 µl PBS und 6 µg markiertes HMGB1-Protein, wie in Beispiel 4 beschrieben dargestellt, bzw. als Negativkontrolle 6 µg FLUOS-Lösung zu den Mz-12-Zellen gegeben. Nach Inkubation für 1 h 30 min bei 37°C und 5 % CO₂ wurden die Deckgläschen in PBS kurz gewaschen und danach auf einem Objektträger eingedeckt. Die Auswertung erfolgte nach ca. 2 h.

### ERGEBNISSE:

Es konnte nach einer Inkubationszeit von 2 h eine Membran-Markierung der Mz-12-Zellen durch die Bindung von Fluorescein-markierten HMGB1-Proteinen an die Zellmembran beobachtet werden.

Der Einsatz des reinen Fluoresceins ergab keine Membran-Positivität, sondern nur eine grünliche diffuse Färbung des Cytoplasmas.

### Beispiel 6: Klonierung von 3 verschiedenen RAGE Transkripten

### METHODIK

Die PCR Amplifikate der Endometriumkarzinomzelllinie (siehe Beispiel 7) wurden in einem 1,2 %igem Agarosegel aufgetrennt und mittels eines sterilem Skalpells aus dem Gel ausgeschnitten. Die Elution der DNA erfolgte mit Hilfe des QIAEX II Systems der Firma Qiagen. Die eluierte DNA wurde nach dem "Ligations Using the pGEM^{®}-T and pGEM^{®}-T Easy Vectors and the 2x Rapid Ligation Buffer" Protokoll der Firma Promega mit Hilfe der T4 DNA Ligase in das pGEM^{®}-T Easy Vektorsystem ligiert und in DH5α *E. coli* nach der Methode von INOUE ET AL. (1990) transformiert. Die Isolierung der Plasmid-DNA erfolgte gemäß dem "QIAprep^{®} Miniprep" Handbuch nach dem "QIAprep Spin Miniprep Kit Protokoll" der Firma QIAGEN. Für die Sequenzierung wurden von der Plasmid-DNA 3 µg DNA im SpeedVac eingedampft. Die Sequenzierung mit dem Primern M13uni (5'-CCCAGTCACGACGTTGTAAAACG-3') (SEQ ID NO. 7) und M13rev (5'-AGCGGATAACAATTTCACACAGG-3') (SEQ ID NO. 8) erfolgte mit dem ABI 377 DNA Sequenzierer (PE-Apllied Biosystems, Weiterstadt).

Die Sequenzen der Plasmid-DNA wurden mit Hilfe des Computerprogramms EditSeq, MegAlign und Seqman (DNAstar) bearbeitet. Der Vergleich der Sequenzen mit bereits bekannten Sequenzen erfolgte über den BLAST Service des National Center for Biotechnology Information (NCBI, USA) unter Verwendung des BLAST Programms (ALTSCHUL ET AL, 1990). Desweiteren wurden die Sequenzen mittels des RepeatMasker Programm auf alle repetitiven Sequenzen überprüft.

### ERGEBNISSE:

Nach PCR und Gelelektrophorese konnten jeweils neben der RAGE-Bande,_die der erwarteten Produktlänge des *RAGE*-Transkriptes entsprach, drei weitere Banden unterschiedlicher Intensität detektiert werden, die sich nach Klonierung und Sequenzierung als RAGE-spezifisch erwiesen und folglich als *sRAGE1, sRAGE2* und *sRAGE3* bezeichnet wurden.

Das 653 bp Fragment wurde als *sRAGE1* bezeichnet. Es entspricht grundsätzlich der Sequenz des *RAGE* Fragments, zusätzlich erfolgte jedoch eine 142 bp Insertion des kompletten Intron 6 des *RAGE* Gens zwischen die Exons 6 und 7 des Transkripts, sowie den Verlust des Exons 10, ersetzt durch die ersten 82 bp des Intron 9 des RAGE-Gens. Durch die Intron 6-Insertion werden, in Anschluß an die letzte komplett durch das Exon 6 kodierte Aminosäure (Tryptophan), inklusive eines neu generierten Stop-Codons 20 neue Aminosäuren kodiert (GEHRWGGPQAHVSTFWKSDP.). Das neue Stop-Codon führt auf Protein-Ebene zu einem Verlust der extrazellulären C'-, der Transmembran-, sowie der zytosolischen Domäne des RAGE-Rezeptors.

Das 511 bp *sRAGE2* Fragment entspricht grundsätzlich der Sequenz des *RAGE* Fragments, das Exons 10 wird jedoch ersetzt durch die ersten 82 bp des Intron 9 des Gens. Hierdurch werden, in Anschluß an die letzte komplett durch das Exon 9 kodierte Aminosäure (Alanin), inklusive eines neu generierten Stop-Codons, 17 neue Aminosäuren kodiert (GEGFDKVREAEDSPQHM.). Auf Protein-Ebene führt dies zu einem Verlust der Transmembran- und der zytosoloischen Domäne des RAGE-Rezeptors.

Das 698 bp *sRAGE3* Fragment entspricht grundsätzlich der Sequenz des *RAGE*Fragments, zusätzlich erfolgte jedoch eine 142 bp Insertion des kompletten Intron 6 des *RAGE*Gens, zwischen die Exons 6 und 7 des Transkripts. Dadurch werden, in Anschluß an die letzte komplett durch das Exon 6 kodierte Aminosäure (Tryptophan), inklusive eines neu generierten Stop-Codons, 20 neue Aminosäuren kodiert (GEHRWGGPQAHVSTFWKSDP.). Das neue Stop-Codon führt auf Protein-Ebene zu einem Verlust der extrazellulären C'-, der Transmembran-, sowie der zytosoloischen Domäne des RAGE-Rezeptors.

### Beispiel 7: RT-PCR zum Nachweis des RAGE-Rezeptors

### METHODIK:

Gewebeproben des Endometriums und Myometriums wurden direkt nach der Entnahme in flüssigem Stickstoff weggefroren. Die RNA-Isolierung aus den Geweben und den Zellen der Endometriumkarzinomzelllinie Mz12 erfolgte nach dem RNeasy Mini Handbook der Firma Qiagen. Dabei wird die RNA an spezifische Säulen gebunden und über spezifische Waschschritte aufgereinigt.

Die cDNA der Gesamt-RNA wurde mit dem Adaptionsprimer AP2 (5'-AAGGATCCGTCGACATC(T)₁₇-3') (SEQ ID NO. 9)_und der M-MLV reversen Transkriptase (Invitrogen, Life Technologies) synthetisiert. Die Detektion des RAGE-Gens erfolgte mit den PrimerRAGE2 up (5'-GATCCCCGTCCCACCTTCTCCTGTAGC-3') (SEQ ID NO. 10) und RAGE2 lo (5'-CACGCTCCTCCTCTTCCTCCTGGTTTTCTG-3') (SEQ ID NO. 11) in 0,2 ml-Cups und einem 20 µl-Reaktionsvolumen. Als Template wurden 125 ng cDNA eingesetzt. Für die Reaktion wurden 0,5 µl der rekombinanten Taq Polymerase (5 U/µl) (Quiagen, Hilden Germany) und 2 µl Quiagen PCR Buffer (10x) eingesetzt. 4 µl 5x Q-Solution (Qiagen), 2 µl dATP, dGTP, dCTP, dTTP (2 mM); sowie 0,4 µl pro Primer (10 µM) wurden zu dem Ansatz dazu gegeben. Die DNA-Amplifikation erfolgte in einem Gradienten-Thermocycler der Firma Eppendorf für 35 Zyklen unter folgenden Bedingungen: 30 sec bei 94°C, 30 sec bei 69°C, und 1 min bei 72°C. Die initiale Denaturierung erfolgte für 2 min bei 95°C sowie eine abschließende Elongation für 10 min bei 72°C.

### ERGEBNISSE:

Die RT-PCR zeigte nach der gelelektrophoretischen Auftrennung sowohl bei 10 Myometriumals auch 10 Endometriumgeweben sowie bei der Endometriumkarzinomzelllinie eine distinkte, spezifische Bande mit einer Größe von 556bp, die der erwarteten Produktlänge des *RAGE-*Transkriptes entsprach. Zusätzlich konnten jeweils drei weitere Banden unterschiedlicher Intensität detektiert werden, die sich nach Klonierung und Sequenzierung als RAGE-spezifisch erwiesen (siehe Beispiel 6).

### SEQUENZEN:

### SEQUENZEN DER RAGE TRANSKRIPTE

sRAGE1 (653 bp) (SEQ ID NO. 4)
*sRAGE2* (511 bp) (SEQ ID NO. 5)
*sRAGE3* (698 bp) (SEQ ID NO. 6)

## Patentansprüche

1. Verwendung von RAGE, sRAGE oder einem Translationsprodukt einer Nukleinsäure gemäß SEQ ID NO. 4, 5 oder 6 für die Herstellung eines Medikamentes zur Behandlung und/oder Prävention von Endometriose.

2. Verwendung von HMGB, einer dafür codierenden Nukleinsäure, eines inbesondere natürlichen Wechselwirkungspartners von HMGB oder einer dafür codierenden Nukleinsäure für die in vitro Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und HMGB-Proteine DNA-Boxen als Bindungsdomänen aufweisen.

3. Verwendung von HMGB, einer dafür codierenden Nukleinsäure, eines insbesondere natürlichen Wechselwirkungspartners von HMGB oder einer dafür codierenden Nukleinsäure als Zielmolekül für die in vitro Entwicklung eines Agens für ein Medikament zur Behandlung und/oder Prävention von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und wobei das Agens aus der Gruppe ausgewählt ist umfassend Antikörper, aus einer Peptidbibliothek durch Phage-Display erhältliche bindende Peptide, Anticaline, Antisense-Moleküle, Aptamere, Spiegelmere und RNAi-Moleküle, wobei das Agens mit HMGB oder mit dem natürlichen Wechselwirkungspartner von HMGB oder mit einer für HMGB codierenden Nukleinsäure oder mit einer für den natürlichen Wechselwirkungspartner von HMGB codierenden Nukleinsäure, insbesondere mit mRNA, genomischer Nukleinsäure oder cDNA für HMGB in Wechselwirkung tritt, und wobei HMGB-Proteine DNA-Boxen als Bindungsdomänen aufweisen.

4. Verwendung von HMGB, einer dafür codierenden Nukleinsäure, eines insbesondere natürlichen Wechselwirkungspartners von HMGB oder einer dafür codierenden Nukleinsäure als Zielmolekül für die in vitro Entwicklung eines Agens für ein diagnostisches Mittel zur Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und wobei das Agens aus der Gruppe ausgewählt ist umfassend Antikörper, aus einer Peptidbibliothek durch Phage-Display erhältliche bindende Peptide, Anticaline, Antisense-Moleküle, Aptamere, Spiegelmere und RNAI-Moleküle, wobei das Agens mit HMGB oder mit dem natürlichen Wechselwirkungspartner von HMGB oder mit einer für HMGB codierenden Nukleinsäure oder mit einer für den natürlichen Wechselwirkungspartner von HMGB codierenden Nukleinsäure, insbesondere mit mRNA, genomischer Nukleinsäure oder cDNA für HMGB in Wechselwirkung tritt, und wobei HMGB-Proteine DNA-Boxen als Bindungsdomänen aufweisen.

5. Verwendung eines an HMGB oder einen insbesondere natürlichen Wechselwirkungspartner von HMGB spezifisch bindenden Polypeptids zur Herstellung eines Medikamentes zur Behandlung und/oder Prävention von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und das Polypeptid ausgewählt ist aus der Gruppe, die aus Antikörpern besteht, und wobei HMGB-Proteine DNA-Boxen als Bindungsdomänen aufweisen.

6. Verwendung eines an HMGB oder einen insbesondere natürlichen Wechselwirkungspartner von HMGB spezifisch bindenden Polypeptids zur Herstellung eines diagnostischen Mittels für die in vitro Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und das Polypeptid ausgewählt ist aus der Gruppe, die aus Antikörpern besteht, und wobei HMGB-Proteine DNA-Boxen als Bindungsdomänen aufweisen.

7. Verwendung eines an HMGB oder insbesondere einen natürlichen Wechselwirkungspartner von HMGB spezifisch bindenden Polypeptids zur in vitro Entwicklung eines Medikamentes zur Behandlung und/oder Prävention von Endometriose und/oder zur in vitro Entwicklung eines diagnostischen Mittels zur Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, und das Polypeptid ausgewählt ist aus der Gruppe, die aus Antikörpern besteht, und wobei HMGB-Proteine DNA-Boxen als Bindungsdomänen aufweisen.

8. Verwendung nach einem der Ansprüche 5 bis 7, **dadurch gekennzeichnet, dass** das Polypeptide ausgewählt ist aus der Gruppe, die aus HMGB spezifisch bindenden Antikörpern besteht.

9. Verwendung einer HMGB oder einen insbesondere natürlichen Wechselwirkungspartner von HMGB spezifisch bindenden Nukleinsäure zur Herstellung eines Medikamentes zur Behandlung und/oder Prävention von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, wobei
die Nukleinsäure ausgewählt ist aus der Gruppe, die aus Aptameren und Spiegelmeren besteht, und wobei HMGB-Proteine DNA-Boxen als Bindungsdomänen aufweisen.

10. Verwendung einer HMGB oder einen insbesondere natürlichen Wechselwirkungspartner von HMGB spezifisch bindenden Nukleinsäure zur Herstellung eines diagnostischen Mittels für die in vitro Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, wobei
die Nukleinsäure ausgewählt ist aus der Gruppe, die aus Aptameren und Spiegelmeren besteht, und wobei HMGB-Proteine DNA-Boxen als Bindungsdomänen aufweisen.

11. Verwendung einer HMGB oder einen insbesondere natürlichen Wechselwirkungspartner von HMGB spezifisch bindenden Nukleinsäure zur in vitro Entwicklung eines Medikamentes zur Behandlung und/oder Prävention von Endometriose und/oder zur in vitro Entwicklung eines diagnostischen Mittels zur Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ ID NO. 4, 5 oder 6, wobei
die Nukleinsäure ausgewählt ist aus der Gruppe, die aus Aptameren und Spiegelmeren besteht, und wobei HMGB-Proteine DNA-Boxen als Bindungsdomänen aufweisen.

12. Verwendung einer mit für HMGB oder für einen insbesondere natürlichen Wechselwirkungspartner von HMGB codierenden Nukleinsäure in Wechselwirkung tretende Nukleinsäure zur Herstellung eines Medikamentes zur Behandlung und/oder Prävention von Endometriose, wobei der natürliche Wechselwirkungspartner ausgewählt ist aus der Gruppe umfassend RAGE, sRAGE und ein Translationsprodukt einer Nukleinsäure gemäß SEQ. ID. No. 4, 5 oder 6, wobei
die in Wechselwirkung tretende Nukleinsäure ein Antisense-Oligonukleotid, ein Ribozym und/oder RNAi ist, wobei
die Wechselwirkung im Falle des Antisense-Oligonukleotids und des Ribozyms eine spezifische Hybridisierung ist und im Falle von RNAi eine spezifische Wechselwirkung ist, und wobei HMGB-Proteine DNA-Boxen als Bindungsdomäne aufweisen.

13. Verwendung einer mit für HMGB oder für einen insbesondere natürlichen Wechselwirkungspartner von HMGB codierenden Nukleinsäure in Wechselwirkung tretende Nukleinsäure zur Herstellung eines diagnostischen Mittels für die in vitro Diagnose von Endometriose, wobei der natürliche Wechselwirkungspartner ausgewählt ist aus der Gruppe umfassend RAGE, sRAGE und ein Translationsprodukt einer Nukleinsäure gemäß SEQ. ID. No. 4, 5 oder 6, wobei
die in Wechselwirkung tretende Nukleinsäure ein Antisense-Oligonukleotid, ein Ribozym und/oder RNAi ist, wobei
die Wechselwirkung im Falle des Antisense-Oligonukleotids und des Ribozyms eine spezifische Hybridisierung ist und im Falle von RNAi eine spezifische Wechselwirkung; und HMGB-Proteine DNA-Boxen als Bindungsdomänen aufweisen.

14. Verwendung einer mit für HMGB oder für einen insbesondere natürlichen Wechselwirkungspartner von HMGB codierenden Nukleinsäure in Wechselwirkung tretende Nukleinsäure zur in vitro Entwicklung eines Medikamentes zur Behandlung und/oder Prävention von Endometriose und/oder zur in vitro Entwicklung eines diagnostischen Mittels zur Diagnose von Endometriose, wobei der natürliche Wechselwirkungspartner ausgewählt ist aus der Gruppe anfassend RAGE, sRAGE und ein Translationsprodukt einer Nukleinsäure gemäß SEQ. ID. No. 4, 5 oder 6, wobei
die in Wechselwirkung tretende Nukleinsäure ein Antisense-Oligonukleotid, ein Ribozym und/oder RNAi ist, wobei
die Wechselwirkung im Falle des Antisense-Oligonukleotids und des Ribozyms eine spezifische Hybridisierung ist und im Falle von RNAi eine spezifische Wechselwirkung, und HMGB-Proteine DNA-Boxen als Bindungsdomänen aufweisen.

15. Verwendung nach einem der Ansprüche 12 bis 14, **dadurch gekennzeichnet, dass** die für HMGB oder für einen insbesondere natürlichen Wechselwirkungspartner von HMGB codierende Nukleinsäure die jeweilige cDNA oder mRNA ist.

16. Verwendung nach einem der Ansprüche 1 bis 15, **dadurch gekennzeichnet, dass** HMGB ausgewählt ist aus der Gruppe, die HMGB1, HMGB2, HMGB3 und SP100-HMG umfasst.

## Claims

1. Use of RAGE, sRAGE or a translation product of a nucleic acid according to SEQ ID NO. 4, 5 or 6 for the manufacture of a medicament for the treatment and/or prevention of endometriosis.

2. Use of HMGB, a nucleic acid coding therefore, an interaction partner of HMGB, in particular a natural interaction partner of HMGB, or a nucleic acid coding therefore for the *in vitro* diagnosis of endometriosis, whereby the natural interaction partner is selected from the group comprising RAGE, sRAGE and a translation product of a nucleic acid according to SEQ ID NO. 4, 5 or 6, and whereby HMGB proteins have DNA boxes as binding domains.

3. Use of HMGB, a nucleic acid coding therefore, an interaction partner of HMGB, in particular a natural interaction partner of HMGB, or a nucleic acid coding therefore as a target molecule for the *in vitro* development of an agent for a medicament for the treatment and/or prevention of endometriosis, whereby the natural interaction partner is selected from the group comprising RAGE, sRAGE and a translation product of a nucleic acid according to SEQ ID NO. 4, 5 or 6, and whereby the agent is selected from the group comprising antibodies, binding peptides obtainable from a peptide library *via* phage display, anticalines, antisense molecules, aptamers, spiegelmers and RNAi molecules, whereby the agent interacts with HMGB or the natural interaction partner of HMGB or a nucleic acid coding for HMGB or a nucleic acid coding for the natural interaction partner of HMGB, in particular with mRNA, genomic nucleic acid or cDNA for HMGB, and whereby HMGB proteins have DNA boxes as binding domains.

4. Use of HMGB, a nucleic acid coding therefore, an interaction partner of HMGB, in particular a natural interaction partner of HMGB, or a nucleic acid coding therefore, as a target molecule for *the in vitro* development of an agent for a diagnostic agent for the diagnosis of endometriosis, whereby the natural interaction partner is selected from the group comprising RAGE, sRAGE and a translation product of a nucleic acid according to SEQ ID NO. 4, 5 or 6, and whereby the agent is selected from the group comprising antibodies, binding peptides obtainable from a peptide library *via* phage display, anticalines, antisense molecules, aptamers, spiegelmers and RNAi molecules, whereby the agent interacts with HMGB or the natural interaction partner of HMGB or a nucleic acid coding for HMGB or a nucleic acid coding for the natural interaction partner of HMGB, in particular with mRNA, genomic nucleic acid or cDNA for HMGB, and whereby HMGB proteins have DNA boxes as binding domains.

5. Use of a polypeptide binding specifically to HMGB or an interaction partner of HMGB, in particular a natural interaction partner of HMGB, for the manufacture of a medicament for the treatment and/or prevention of endometriosis, whereby the natural interaction partner is selected from the group comprising RAGE, sRAGE and a translation product of a nucleic acid according to SEQ ID NO. 4, 5 or 6, and whereby the polypeptide is selected from the group consisting of antibodies, and whereby HMGB proteins have DNA boxes as binding domains.

6. Use of a polypeptide specifically binding to HMGB or an interaction partner of HMGB, in particular a natural interaction partner of HMGB, for the manufacture of a diagnostic agent for the *in vitro* diagnosis of endometriosis, whereby the natural interaction partner is selected from the group comprising RAGE, sRAGE and a translation product of a nucleic acid according to SEQ ID NO. 4, 5 or 6, and whereby the polypeptide is selected from the group consisting of antibodies, and whereby HMGB proteins have DNA boxes as binding domains.

7. Use of a polypeptide specifically binding to HMGB or an interaction partner of HMGB, in particular a natural interaction partner of HMGB, for the *in vitro* development of a medicament for the treatment and/or prevention of endometriosis and/or for the *in vitro* development of a diagnostic agent for the diagnosis of endometriosis, whereby the natural interaction partner is selected from the group comprising RAGE, sRAGE and a translation product of a nucleic acid according to SEQ ID NO. 4, 5 or 6, and whereby the polypeptide is selected from the group consisting of antibodies, and whereby HMGB proteins have DNA boxes as binding domains.

8. The use according to any of claims 5 to 7, **characterised in that** the polypeptide is selected from the group consisting of antibodies binding specifically to HMGB.

9. Use of a nucleic acid specifically binding to HMGB or to an interaction partner of HMGB, in particular a natural interaction partner of HMGB, for the manufacture of a medicament for the treatment and/or prevention of endometriosis, whereby the natural interaction partner is selected from the group comprising RAGE, sRAGE and a translation product of a nucleic acid according to SEQ ID NO. 4, 5 or 6, whereby
the nucleic acid is selected from the group consisting of aptamers and spiegelmers, and whereby HMGB proteins have DNA boxes as binding domains.

10. Use of a nucleic acid specifically binding to HMGB or to an interaction partner of HMGB, in particular a natural interaction partner of HMGB, for the manufacture of a diagnostic agent for the *in vitro* diagnosis of endometriosis, whereby the natural interaction partner is selected from the group comprising RAGE, sRAGE and a translation product of a nucleic acid according to SEQ ID NO. 4, 5 or 6, whereby
the nucleic acid is selected from the group consisting of aptamers and spiegelmers, and whereby HMGB proteins have DNA boxes as binding domains.

11. Use of a nucleic acid specifically binding to HMGB or to an interaction partner of HMGB, in particular a natural interaction partner of HMGB, for *the in vitro* development of a medicament for the treatment and/or prevention of endometriosis and/or for the *in vitro* development of a diagnostic agent for the diagnosis of endometriosis, whereby the natural interaction partner is selected from the group comprising RAGE, sRAGE and a translation product of a nucleic acid according to SEQ ID NO. 4, 5 or 6, whereby
the nucleic acid is selected from the group consisting of aptamers and spiegelmers, and whereby HMGB proteins have DNA boxes as binding domains.

12. Use of a nucleic acid interacting with a nucleic acid coding for HMGB or for an interaction partner of HMGB, in particular a natural interaction partner of HMGB, for the manufacture of a medicament for the treatment and/or prevention of endometriosis, whereby the natural interaction partner is selected from the group comprising RAGE, sRAGE and a translation product of a nucleic acid according to SEQ ID NO. 4, 5 or 6, whereby
the interacting nucleic acid is an antisense oligonucleotide, a ribozyme and/or RNAi, whereby
the interaction, in the case of the antisense oligonucleotide and the ribozyme, is a specific hybridisation and, in the case of RNAi, is a specific interaction, and whereby HMGB proteins have DNA boxes as binding domains.

13. Use of a nucleic acid interacting with a nucleic acid coding for HMGB or for an interaction partner of HMGB, in particular a natural interaction partner of HMGB, for the manufacture of a diagnostic agent for *the in vitro* diagnosis of endometriosis, whereby the natural interaction partner is selected from the group comprising RAGE, sRAGE and a translation product of a nucleic acid according to SEQ ID NO. 4, 5 or 6, whereby
the interacting nucleic acid is an antisense oligonucleotide, a ribozyme and/or RNAi, whereby
the interaction, in the case of the antisense oligonucleotide and the ribozyme, is a specific hybridisation and, in the case of RNAi, is a specific interaction; and whereby HMGB proteins have DNA boxes as binding domains.

14. Use of a nucleic acid interacting with a nucleic acid coding for HMGB or for an interaction partner of HMGB, in particular a natural interaction partner of HMGB, for the *in vitro* development of a medicament for the treatment and/or prevention of endometriosis and/or for the *in vitro* development of a diagnostic agent for the diagnosis of endometriosis, whereby the natural interaction partner is selected from the group comprising RAGE, sRAGE and a translation product of a nucleic acid according to SEQ ID NO. 4, 5 or 6, whereby
the interacting nucleic acid is an antisense oligonucleotide, a ribozyme and/or RNAi, whereby
the interaction, in the case of the antisense oligonucleotide and the ribozyme, is a specific hybridisation and, in the case of RNAi, is a specific interaction, and whereby HMGB proteins have DNA boxes as binding domains.

15. The use according to any of claims 12 to 14, **characterised in that** the nucleic acid coding for HMGB or for an interaction partner of HMGB, in particular a natural interaction partner of HMGB, is the respective cDNA or mRNA.

16. The use according to any of claims 1 to 15, **characterised in that** HMGB is selected from the group comprising HMGB1, HMGB2, HMGB3 and SP 100-HMG.

## Revendications

1. Utilisation de RAGE, sRAGE ou d'un produit de traduction d'un acide nucléique selon SEQ ID N° 4, 5 ou 6 pour la production d'un médicament pour le traitement et/ou la prévention de l'endométriose.

2. Utilisation de HMGB, d'un acide nucléique codant pour celui-ci, en particulier d'un partenaire d'interaction naturel de HMGB ou d'un acide nucléique codant pour celui-ci pour le diagnostic *in vitro* de l'endométriose, le partenaire d'interaction naturel étant choisi dans le groupe comprenant RAGE, sRAGE et un produit de traduction d'un acide nucléique selon SEQ ID N° 4, 5 ou 6, et les protéines HMGB présentant des boîtes d'ADN comme domaines de liaison.

3. Utilisation de HMGB, d'un acide nucléique codant pour celui-ci, en particulier d'un partenaire d'interaction naturel de HMGB ou d'un acide nucléique codant pour celui-ci comme molécule cible pour le développement in *vitro* d'un agent pour un médicament pour le traitement et/ou la prévention de l'endométriose, dans laquelle le partenaire d'interaction naturel est choisi dans le groupe comprenant RAGE, sRAGE et un produit de traduction d'un acide nucléique selon SEQ ID N° 4, 5 ou 6 et dans laquelle l'agent est choisi dans le groupe comprenant des anticorps, des peptides de liaison obtenus par phage-display dans une bibliothèque de peptides, des anticalines, des molécules anti-sens, des aptamères, des aptamères miroirs "spiegelmers" et des molécule d'ARNi, l'agent interagissant avec HMGB ou avec le partenaire d'interaction naturel de HMGB ou avec l'acide nucléique codant pour HMGB ou avec un acide nucléique codant pour le partenaire d'interaction naturel de HMGB, en particulier avec l'ARNm, l'acide nucléique génomique ou l'ADNc de HMGB et dans laquelle les protéines HMGB présentent des boîtes d'ADN comme domaines de liaison.

4. Utilisation de HMGB, d'un acide nucléique codant pour celui-ci, en particulier d'un partenaire d'interaction naturel de HMGB ou d'un acide nucléique codant pour celui-ci comme molécule cible pour le développement in *vitro* d'un agent pour un moyen de diagnostic pour le diagnostic de l'endométriose, dans lequel le partenaire d'interaction naturel est choisi dans le groupe comprenant RAGE, sRAGE et un produit de traduction d'un acide nucléique selon SEQ ID N° 4, 5 ou 6 et dans laquelle l'agent est choisi dans le groupe comprenant des anticorps, des peptides de liaison obtenus par phage-display dans une bibliothèque de peptides, des anticalines, des molécules anti-sens, des aptamères, des aptamères miroirs "spiegelmers" et des molécule d'ARNi, l'agent interagissant avec HMGB ou avec le partenaire d'interaction naturel de HMGB ou avec l'acide nucléique codant pour HMGB ou avec un acide nucléique codant pour le partenaire d'interaction naturel de HMGB, en particulier avec l'ARNm, l'acide nucléique génomique ou l'ADNc de HMGB et dans laquelle les protéines HMGB présentent des boîtes d'ADN comme domaines de liaison.

5. Utilisation d'un polypeptide se liant spécifiquement à HMGB ou en particulier à un partenaire de liaison naturel de HMGB pour la production d'un médicament pour le traitement et/ou la prévention de l'endométriose, le partenaire d'interaction naturel étant choisi dans le groupe comprenant RAGE, sRAGE et un produit de traduction d'un acide nucléique selon SEQ ID N° 4, 5 ou 6 et le polypeptide étant choisi dans le groupe constitué d'anticorps, et dans laquelle les protéines HMGB présentent des boîtes d'ADN comme domaines de liaison.

6. Utilisation d'un polypeptide se liant spécifiquement à HMGB ou en particulier à un partenaire de liaison naturel de HMGB pour la production d'un moyen de diagnostic pour le diagnostic *in vitro* de l'endométriose, le partenaire d'interaction naturel étant choisi dans le groupe comprenant RAGE, sRAGE et un produit de traduction d'un acide nucléique selon SEQ ID N° 4, 5 ou 6 et le polypeptide étant choisi dans le groupe constitué d'anticorps, et dans laquelle les protéines HMGB présentent des boîtes d'ADN comme domaines de liaison.

7. Utilisation d'un polypeptide se liant spécifiquement à HMGB ou en particulier à un partenaire de liaison naturel de HMGB pour le développement *in vitro* d'un médicament pour le traitement et/ou la prévention de l'endométriose et/ou pour le développement *in vitro* d'un moyen de diagnostic pour le diagnostic de l'endométriose, le partenaire d'interaction naturel étant choisi dans le groupe comprenant RAGE, sRAGE et un produit de traduction d'un acide nucléique selon SEQ ID N° 4, 5 ou 6 et le polypeptide étant choisi dans le groupe constitué d'anticorps, et dans laquelle les protéines HMGB présentent des boîtes d'ADN comme domaines de liaison.

8. Utilisation selon l'une quelconque des revendications 5 à 7, **caractérisée en ce que** le polypeptide est choisi dans le groupe constitué d'anticorps se liant spécifiquement à HMGB.

9. Utilisation d'un acide nucléique se liant spécifiquement à HMGB ou en particulier à un partenaire d'interaction naturel de HMGB pour la production d'un médicament pour le traitement et/ou la prévention de l'endométriose, le partenaire d'interaction naturel étant choisi dans le groupe comprenant RAGE, sRAGE et un produit de traduction d'un acide nucléique selon SEQ ID N° 4, 5 ou 6, l'acide nucléique étant choisi dans le groupe comprenant les apatmères et les aptamères miroirs "spiegelmers" et dans laquelle les protéines HMGB présentent des boîtes d'ADN comme domaines de liaison.

10. Utilisation d'un acide nucléique se liant spécifiquement à HMGB ou en particulier à un partenaire d'interaction naturel de HMGB pour la production d'un moyen de diagnostic pour le diagnostic *in vitro* de l'endométriose, le partenaire d'interaction naturel étant choisi dans le groupe comprenant RAGE, sRAGE et un produit de traduction d'un acide nucléique selon SEQ ID N° 4, 5 ou 6,
l'acide nucléique étant choisi dans le groupe comprenant les apatmères et les aptamères miroirs "spiegelmers" et dans laquelle les protéines HMGB présentent des boîtes d'ADN comme domaines de liaison.

11. Utilisation d'un acide nucléique se liant spécifiquement à HMGB ou en particulier à un partenaire d'interaction naturel de HMGB pour le développement *in vitro* d'un médicament pour le traitement et/ou la prévention de l'endométriose et/ou pour le développement *in vitro* d'un moyen de diagnostic pour le diagnostic de l'endométriose, le partenaire d'interaction naturel étant choisi dans le groupe comprenant RAGE, sRAGE et un produit de traduction d'un acide nucléique selon SEQ ID N° 4, 5 ou 6,
l'acide nucléique étant choisi dans le groupe comprenant les apatmères et les aptamères miroirs "spiegelmers" et dans laquelle les protéines HMGB présentent des boîtes d'ADN comme domaines de liaison.

12. Utilisation d'un acide nucléique interagissant avec l'acide nucléique codant pour HMGB ou en particulier un partenaire d'interaction naturel de HMGB pour la production d'un médicament pour le traitement et/ou la prévention de l'endométriose, le partenaire d'interaction naturel étant choisi dans le groupe comprenant RAGE, sRAGE et un produit de traduction d'un acide nucléique selon SEQ ID N° 4, 5 ou 6,
l'acide nucléique interagissant étant un oligonucléotide anti-sens, un ribozyme et/ou un ARNi,
dans laquelle l'interaction, dans le cas de l'oligonucléotide anti-sens et du ribozyme est une hybridation spécifique et dans le cas de l'ARNi, est une interaction spécifique et dans laquelle les protéines HMGB présentent des boîtes d'ADN comme domaines de liaison.

13. Utilisation d'un acide nucléique interagissant avec l'acide nucléique codant pour HMGB ou en particulier un partenaire d'interaction naturel de HMGB pour la production d'un moyen de diagnostic pour le diagnostic *in vitro* de l'endométriose, le partenaire d'interaction naturel étant choisi dans le groupe comprenant RAGE, sRAGE et un produit de traduction d'un acide nucléique selon SEQ ID N° 4, 5 ou 6,
l'acide nucléique interagissant étant un oligonucléotide anti-sens, un ribozyme et/ou un ARNi,
dans laquelle l'interaction, dans le cas de l'oligonucléotide anti-sens et du ribozyme est une hybridation spécifique et dans le cas de l'ARNi, est une interaction spécifique et dans laquelle les protéines HMGB présentent des boîtes d'ADN comme domaines de liaison.

14. Utilisation d'un acide nucléique interagissant avec l'acide nucléique codant pour HMGB ou en particulier un partenaire d'interaction naturel de HMGB pour le développement *in vitro* d'un médicament pour le traitement et/ou la prévention de l'endométriose et/ou pour le développement *in vitro* d'un moyen de diagnostic pour le diagnostic de l'endométriose, le partenaire d'interaction naturel étant choisi dans le groupe comprenant RAGE, sRAGE et un produit de traduction d'un acide nucléique selon SEQ ID N° 4, 5 ou 6,
l'acide nucléique interagissant étant un oligonucléotide anti-sens, un ribozyme et/ou un ARNi,
dans laquelle l'interaction, dans le cas de l'oligonucléotide anti-sens et du ribozyme est une hybridation spécifique et dans le cas de l'ARNi, est une interaction spécifique et dans laquelle les protéines HMGB présentent des boîtes d'ADN comme domaines de liaison.

15. Utilisation selon l'une quelconque des revendication 12 à 14, **caractérisée en ce que** l'acide nucléique codant pour HMGB ou en particulier pour un partenaire d'interaction naturel de HMGB est l'ADNc ou l'ARNm respectif.

16. Utilisation selon l'une quelconque des revendication 1 à 15, **caractérisée en ce que** HMGB est choisi dans le groupe comprenant HMGB1, HMGB2, HMGB3 et SP100-HMG.
